# EUROPEAN PATENT SPECIFICATION

(11) **EP 2 347 776 B1**
(45) Date of publication and mention of the grant of the patent: **26.04.2017**
(21) Application number: 11163734.4
(22) Date of filing: 14.12.2006
(51) Int. Cl.: A61L 31/10, A61L 31/16, A61L 27/34, A61L 27/54

(54) **Nanoparticle releasing medical devices**
NANOPARTIKEL FREISETZENDE MEDIZINISCHE GERÄTE
DISPOSITIFS MÉDICAUX LIBÉRANT DES NANOPARTICULES

(30) Priority: 22.12.2005 US 317837
(43) Date of publication of application: 27.07.2011
(62) Divisional of application: 06851482.7
(73) Proprietor: Abbott Cardiovascular Systems Inc., Santa Clara, CA 95054 (US)
(72) Inventor: Hossainy, Syed Faiyaz Ahmed, Fremont, CA 94555 (US); Ludwig, Florian Niklas, Mountain View, CA 94041 (US); Sridharan, Srinivasan, Mendham, NJ 07945 (US)
(74) Representative: Keen, Celia Mary

(56) References cited:
- WO-A-2005/115496
- WO-A2-03/065996
- WO-A2-2004/069169
- US-A1- 2005 119 723
- US-A1- 2005 181 015
- US-A1- 2005 209 680
- US-B1- 6 808 535

## Description

### Field of the Invention

This invention is generally related to nanoparticle releasing medical devices, such as drug releasing vascular stents.

### Description of the State of the Art

Stents are used not only as a mechanical intervention of vascular conditions but also as a vehicle for providing biological therapy. As a mechanical intervention, stents act as scaffoldings, functioning to physically hold open and, if desired, to expand the wall of the passageway. Typically, stents are capable of being compressed, so that they can be inserted through small vessels via catheters, and then expanded to a larger diameter once they are at the desired location. Examples in patent literature disclosing stents which have been applied in PTCA (Percutaneous Transluminal Coronary Angioplasty) procedures include stents illustrated in U.S. Patent No. 4,733,665 issued to Palmaz, U.S. Patent No. 4,800,882 issued to Gianturco, and U.S. Patent No. 4,886,062 issued to Wiktor.

Biological therapy can be achieved by medicating the stents. Medicated stents provide for the local administration of a therapeutic substance at the diseased site. In order to provide an efficacious concentration to the treated site, systemic administration of such medication often produces adverse or toxic side effects on the patient. Local delivery is a preferred method of treatment in that smaller total levels of medication are administered in comparison to systemic dosages, but are concentrated at a specific site. Local delivery thus produces fewer side effects and achieves more favorable results.

WO 2004/069169 A2 discloses an implantable or insertable medical device with a biodegradable hydrogel coating comprising nanocapsules, the nanocapsules comprising a therapeutic agent, a polyelectrolyte multilayer shell encapsulating said therapeutic agent and a tissue specific ligand attached to an outer surface of said polyelectrolyte multilayer shell, wherein said nanocapsules are released upon degradation of said biodegradable coating layer.
The polyelectrolytes may be biodegradable. Moreover,
the nanocapsule can be rendered paramagnetic to facilitate its release in the body by adding metallic particles in the capsule (e.g iron). Once the nanocapsules are released, they release the therapeutic agent even if the medical device is removed.

In many patients, especially diabetic patients, stentable lesions are focal manifestations of widespread vascular disease. The advent of drug eluting stents has brought relief from restenosis of the treated lesion, but leaves progression of regional vascular disease unaddressed.

The embodiments described below address the above-identified problems.

### SUMMARY

The present invention provides nanoparticles and medical devices (such as, for example, a stent) containing the nanoparticles, as defined in the claims. The nanopartiles include a bioactive agent and further include a matrix material. The nanoparticles can be loaded onto and released from the medical device (e.g., stent) via a porous matrix, a channeled surface, a depot structure, or a stent with nanoporous or micro-depot surface. Alternatively, the nanoparticles can be included in a coating on a medical device (e.g., a drug-delivery stent coating) and released therefrom upon implantation of the medical device. In some configurations, the nanoparticles have a shell enclosing a volume, or the nanoparticles may include a porous material which can be deployment from a nano-, micro-, or macroporous structure on the stent surface, e.g., carbon, metal, ceramic, plastic or polymeric porous surface. Alternatively, nanoparticles can be released from micro-depots in a stent surface, which can be a carbon, metal, ceramic, plastic or polymeric surface. Such depots may be laser-drilled into the surface.

In some embodiments, the device (e.g., stent) or a portion thereof can be biodegradable. Nanoparticles may be embedded within the matrix of the device (e.g., biodegradable polymeric stent matrix) and released upon degradation of the device. In these embodiments, nanoparticles either have degradation time scales longer than the polymeric matrix or do not begin to degrade until release from the matrix. One example of this nanoparticle degradation pattern is that the nanoparticles are degraded by enzymatic degradation and the matrix will shield the nanoparticles from degradation until release.

Some examples of bioactive agents in the nanoparticles include, but are not limited to, paclitaxel, docetaxel, estradiol, nitric oxide donors, super oxide dismutases, super oxide dismutases mimics, 4-amino-2,2,6,6-tetramethylpiperidine-1-oxyl (4-amino-TEMPO), tacrolimus, dexamethasone, rapamycin, rapamycin derivatives, 40-*O*-(2-hydroxy)ethyl-rapamycin (everolimus), 40-*O*-(3-hydroxy)propyl-rapamycin, 40-*O*-[2-(2-hydroxy)ethoxy]ethyl-rapamycin, and 40-*O*-tetrazole-rapamycin 40-epi-(N1-tetrazolyl)-rapamycin (ABT-578), pimecrolimus, imatinib mesylate, midostaurin, clobetasol, bioactive RGD, CD-34 antibody, abciximab (REOPRO), progenitor cell capturing antibody, prohealing drugs, prodrugs thereof, co-drugs thereof, or a combination thereof.

The medical device including nanoparticles described herein can be used to treat, prevent, or ameliorate a vascular medical condition.

### DETAILED DESCRIPTION

The present invention provides nanoparticles and medical devices (such as, for example, a stent) containing the nanoparticles, as defined in the claims. The nanoparticles include a bioactive agent and further include a matrix material. The nanoparticles can be loaded onto and released from the medical device via a porous matrix, a channeled surface, a depot structure, or a stent coated with porous or micro-depot surface. Alternatively, the nanoparticles can be included in a coating on a medical device (e.g., a drug-delivery stent coating) and released therefrom upon implantation of the medical device. It is to be understood that medical devices contemplated hereunder include but are not intended to be limited to, implantable devices comprising any suitable medical substrate that can be implanted in a human or veterinary patient.

The nanoparticles can be released from stent surface by several mechanisms. For example, the nanoparticles can be polymeric particles and can be released from a polymeric stent coating where the polymeric stent coating degrades on a time scale faster than the polymeric nanoparticles. Nanoparticles may be loaded pre-deployment into and released post-deployment from a porous structure on the stent surface, e.g., carbon, metal, ceramic, plastic or polymeric porous surface. The porosity can be on a nanoscale or a larger scale. In some embodiments, nanoparticles can be released from micro-depots in a stent surface, which can be a carbon, metal, ceramic, plastic or polymeric surface. Such depots may be laser-drilled into the surface.

In some embodiments, the device (e.g., stent) or a portion thereof can be biodegradable. Nanoparticles may be embedded within a matrix comprising at least a portion of the device (e.g., biodegradable polymeric stent matrix) and released upon biodegradation, absorption or erosion of the device or parts thereof. Biodegradation, absorption or erosion are terms that are used interchangeably unless otherwise indicated. In these embodiments, nanoparticles either have degradation time scales longer than the polymeric matrix or do not begin to degrade until release from the matrix, which can be achieved if the matrix shields the particles from degradation. One example of this nanoparticle degradation pattern is that if nanoparticles are degraded by enzymatic degradation, the matrix can shield the nanoparticles from degradation until release. In some embodiments, the time scale of degradation can be the same or generally the same. In yet other embodiments, the nanoparticles have degradation time scales shorter than the polymeric matrix. In such embodiments, the nanoparticles can impart mechanical or material functionality to the device, such as strength, elasticity, etc.

In some embodiments, the nanoparticles can be included in a polymeric coating. The coating can be biodegradable, and the nanoparticles have a degradation time scale longer than the coating or do not begin to degrade until after released from the coating. In some embodiments, the coating can be non-biodegradable or biostable, and the nanoparticles do not begin to degrade until after released from the coating. In yet other embodiments, the time scale of degradation of the particles is slower or shorter than the degradation rate of the coating. In some embodiments, rate of degradation of the coating and the particle can be the same or generally the same.

### Nanoparticles

In one embodiment, the nanoparticles include a bioactive agent such as, for example, a drug, protein, peptide, and the like agents as described hereinbelow, the content of which, as used herein, is sometimes referred to as "payload", and a matrix material. The matrix material may be porous. Alternatively, the nanoparticles may comprise a shell encapsulating a volume including a drug. The matrix or shell material can comprise polymeric, ceramic, metallic or bioglass materials, or combinations thereof. The matrix or shell can be biodegradable or non-degradable and can include one or more of the biocompatible materials, e.g. polymers, described herein. The biocompatible polymer can be a random or block copolymer.

In some embodiments, these materials may be layered to tailor release kinetics to specific applications. For example, a nanoparticle having a drug-loaded polymeric matrix may be sputter-coated with a biodegradable metal to allow for delayed and timed release of the drug or to shield the polymeric matrix from degradation for some time interval after implantation.

In some embodiments, the particles can be modified to impart mechanical and biological properties. For example, surfaces of the particles can be modified by grafting of polymers, peptides or proteins to enhance biocompatibility of the particles. In one embodiment, the grafted molecules (e.g., polyethylene glycol) can serve to evade immune-response or to target the payload carriers (e.g., a peptide with affinity to a vasculature surface molecule).

In some embodiments, the particles can include biopolymers for higher uptake and partitioning in the vessel wall and lesion or for adhesion to vascular tissue of the particles. Such biopolymers can be, for example, chitosan, silk elastin, poly(acrylic acid) (PAA), lectin-conjugated polymers, lipid- or cholesterol-conjugated polymers or co-polymers and combinations thereof. In some embodiments, the particles can include antibodies to receptors found on vascular cells such as endothelial cells or antibodies to proteins of the subendothelial matrix or combinations thereof. In one embodiment, the particles include poly(ester amide) (PEA). Conjugation of lectin, cholesterol or lipid to a polymer can be readily achieved via the reactive/functional groups on the lectin, cholesterol and polymer molecules, such as hydroxyl, carboxyl, amino, thiol, and aldehyde groups, with or without a linker, using conventional coupling chemistry, e.g., Sharma et al. J. Antimicrob. Chemother, 2004; 54: 761-766.

In some other embodiments, the particles can include a metallic matrix or shell. Such matrix or shell can include, for example, manganese (Mn), gold (Au), iron, iron oxides, rare earth materials or combinations thereof. In some embodiments, the particles can also include ceramic and/or biodegradable glass matrix material or shell. For example, bioglass can be formed of a biocompatible and/or inorganic material such as phosphorous oxide, silicon oxide, calcium oxide, or other inorganic materials. Examples of nanoparticles or nanosperes formed of biodegradable glass are described in U.S. Patent No. 6,328,990 and Qiu et al., Annals of the New York Academy of Sciences 974:556-564 (2002). Some examples of ceramic nanoparticles and methods of forming the ceramic nanoparticles are described in Roy, I., et al., J. Am. Chem. Soc. 2003, 125, 7860-7865. Some examples of metallic nanoparticles and methods of forming metallic nanoparticles are described in Sakai and Alexandridis, "Single-Step Synthesis and Stabilization of Metal Nanoparticles in Aqueous Pluronic Block Copolymer Solutions at Ambient Temperature" in Langmuir, 2004; Rao, C.N.R., et al., "Metal nanoparticles, nanowires, and carbon nanotubes" in Pure Appl. Chem., Vol. 72, Nos. 1-2:21-33 (2000); and Kim, et al., "Size-monodisperse metal nanoparticles via hydrogen-free spray pyrolysis" in Advanced Materials, 14(7):528-521 (2002).

In some embodiments, the nanoparticles described herein can be micelles (e.g., polymer micelles), liposomes, polyliposomes, polymerosomes, or membrane vesicles with a membrane that includes a polymerosomes. The term "polymerosome" refers to an amphiphilic block co-polymer.

In some embodiments, the nanoparticles are spherical or quasi-spherical nanoparticles formed of a polymer encapsulating a drug. Typically, nanoparticles having the characteristic length (e.g., the diameter) between about 0.001 µm (1 nm) and about 500 µm (e.g., between about 0.12 µm and 5.0 µm) can be utilized. When the stent is in contact with body fluids, the polymer can swell and/or hydrolyze, thus releasing the drug.

In some embodiments, these nanoparticles can be coated onto the surface of a medical device (e.g., stent), with or without a biocompatible polymer(s), and then top coated with one or more biocompatible polymers. In some embodiments, a primer layer of a biocompatible polymer(s) can be coated between the layer of the nanoparticles and the surface of the device.

The nanoparticles including a polymeric matrix can be formed in a separate procedure, followed by suspending the nanoparticles in organic phase such as an organic solvent, for example methanol, or a solution of a biocompatible polymer such as poly(ethylene-co-vinyl alcohol) (EVAL™). The suspension can be then applied onto the stent to form the drug layer or the drug-polymer layer, respectively. The mass ratio between the nanoparticles and the polymer in the suspension can be within a range of between about 1:2 and 1:10.

In some embodiments, the nanoparticles are formed of a polymeric material that can be made according to one of the methods described below.

### 1. The Double Emulsion Method

One method of fabricating the nanoparticles according to an embodiment of the present invention is the double emulsion technique. This procedure can be used when it is desirable to encapsulate water soluble drugs, peptides or proteins. For the purposes of the present invention, the term "water soluble" is defined as small molecule drugs, peptides, oligonucleotides, plasmids, or proteins that can form aqueous solutions having concentrations within a range between about 3 and 20 mass %. Examples of drugs that can be used include heparin, hyaluronic acid, L-arginine, D-arginine, polymers and/or oligomers of L-arginine or D-arginine, gene encoding vascular endothelial growth factor (VEGF) and its isoforms, and gene encoding nitric oxide synthase (NOS) and its isoforms.

An example of a peptide suitable for incorporation in the nanoparticles is poly(L-arginine), poly(D-arginine) or a combination thereof. In some embodiments, the peptide is poly(D,L-arginine), poly(L-lysine), poly(D-lysine), poly(*δ*-guanidino-*α-*aminobutyric acid), or combinations thereof. Those having ordinary skill in the art may choose to use other appropriate drugs, peptides or proteins, if desired.

As a first step, a solution of an encapsulating polymer in a suitable organic solvent can be prepared (solution I). The concentration of the encapsulating polymer in solution I can be between about 2.0% w/v and about 20% w/v. One example of a suitable encapsulating polymer is poly(L-glycolic acid) (PLGA). In some embodiments, the polymer can be poly(D-lactic acid) (PDLA), poly(L-lactic acid) (PLLA), poly(L-lactide), poly(D,L-lactide), polyglycolide, poly(butylene terephtalate-co-ethylene glycol)(PBT-PEG), poly(ethylene-co-vinyl alcohol) (EVAL), other vinyl polymers such as poly(vinyl acetate) (PVA), acrylic polymers such as poly(butyl methacrylate) (PBMA) or poly(methyl methacrylate) (PMMA), polyurethanes, poly(caprolactone), polyanhydrides, polydiaxanone, polyorthoesters, polyamino acids, poly(trimethylene carbonate), and combinations thereof. Examples of organic solvents that can be used include methylene chloride, cyclooctane, cyclohexane, cycloheptane, para-xylene, dimethylformamide, dimethylsulfoxide, chloroform, dimethylacetamide, or combinations thereof.

As a second step, an aqueous solution of a drug can be prepared (solution II) by dissolving the drug in de-ionized water. The solution can be plain or buffered. Optionally, viscosity enhancing agents and/or drug stabilizing agents such as poly(vinylpyrrolidone) or carboxymethylcellulose can be added to the solution II in the amount of about 0.01% w/v to about 0.5% w/v. Excipients (inert substances used as diluents or vehicles for a drug) and drug stabilizing agents may optionally be added to solution II.

As a third step, the organic phase (solution I) can be combined with the aqueous phase (solution II) and the blend of the two solutions is treated by ultrasound (sonicated) according to techniques known to those having ordinary skill in the art to yield a microfine water-in-oil (W-O) emulsion. Standard sonication equipment can be used. Alternatively, solution I can be vigorously stirred or vortexed while solution II is slowly added to solution I also resulting in the W-O emulsion. The emulsion is comprised of the aqueous phase 1 dispersed in the organic phase 2.

As a fourth step, an aqueous solution of an emulsifier (surfactant) can be prepared (solution III) by dissolving the emulsifier in de-ionized water. The concentration of the emulsifier can be within a range of between 0.01% w/v and 0.5% w/v. One example of a suitable emulsifier is poly(vinyl alcohol) (PVOH). Examples of the alternative emulsifiers that can be used include albumin (either bovine or human serum), gelatin, lipophilic emulsifiers such as PLURONIC or TETRONIC, or combinations thereof. PLURONIC is a trade name of poly(ethylene oxide-co-propylene oxide). TETRONOC is a trade name of a family of non-ionic tetrafunctional block-copolymer surfactants. PLURONIC and TETRONIC are available from, e.g., BASF Corp. of Parsippany, New Jersey.

Solution III can be vigorously stirred while the W-O emulsion is slowly added to solution III to produce a double emulsion, which is referred to as water-oil-water (W-O-W) emulsion. The double emulsion includes nanoparticles dispersed in the aqueous phase. The nanoparticles include an encapsulating polymer and a agent (e.g., a drug) encapsulated within the encapsulating polymer.

As the fifth step, the double emulsion can then be stirred in excess water to extract the organic solvent present in the organic phase inside the nanoparticles. Instead of water, an aqueous solution of a water-soluble organic substance such as *iso-*propanol can be used. In some embodiments, the organic solvent can be removed from the organic phase by evaporation, optionally under suitable vacuum. The hardened nanoparticles can then be collected by filtration, sieving or centrifugation and lyophilized to form a free-flowing dry powder of nanoparticles.

### 2. The Water-in-Oil Emulsion Method

Another method of fabricating the nanoparticles according to an embodiment of the present invention includes preparing a water-in-oil emulsion followed by evaporation of solvent.

As a first step, a solution containing about 10 mass % of an encapsulating polymer in an organic solvent can be prepared. One example of the encapsulating polymer that can be used according to this technique is cellulose acetate phthalate (CAP) available from, e.g., FMC Biopolymers Co. of Philadelphia, Pennsylvania under the trade name AQUACOAT. Those having ordinary skill in the art will select other suitable encapsulating polymers, if desired. A drug, for example, everolimus, trapidil, or cisplatin can be dispersed in the CAP solution, to make a drug-polymer dispersion which can contain about 5 mass % of the drug. Everolimus is the nonproprietary name of 40-O-(2-hydroxy)ethyl-rapamycin, which is available from Novartis.

As a second step, a liquid paraffin can be combined with a suitable surfactant, and the blend can be vigorously stirred. The paraffin-surfactant composition can include about 1 mass % of the surfactant. Sorbitan oleate is one example of a suitable surfactant, but those having ordinary skill in the art can select other appropriate surfactants if necessary. Sorbitan oleate is available form ICI Americas, Inc. of Bridgewater, New Jersey under a trade name SPAN 80.

As a third step, the drug-polymer solution can be added to the paraffin-based composition and the solvent is allowed to evaporate for about 24 hours at a temperature of about 30°C. As a result, the nanoparticles are formed, collected, washed with, e.g., ether, and dried at room temperature for about 24 hours.

### 3. The Spray-Drying Method

Yet another method of fabricating the nanoparticles according to an embodiment of the present invention is the spray drying technique. This procedure can be used when it is desirable to encapsulate drugs soluble in organic solvents. According to this technique, the solution comprising a drug and an encapsulating polymer can be dissolved in an appropriate organic solvent in which both the drug and the encapsulating polymer are soluble. One example of a suitable solvent can be methylene chloride. The solution can then be spray dried according to a method known to those having ordinary skill in the art. As a result, nanoparticles are formed comprising the drug encapsulated in the polymer.

One variation of the spray-drying methods can be used with drugs which are water-soluble but not soluble in common organic solvents. Such drugs can be first formulated as lyophilized powder. The drug powder can be suspended in a polymer phase comprising a suitable encapsulating polymer dissolved in a volatile organic solvent such as methylene chloride. The suspension can then be spray dried to produce the nanoparticles containing the drug.

### 4. The Cryogenic Method

Another method of fabricating the nanoparticles according to an embodiment of the present invention is the cryogenic technique. This procedure can be used for processing sensitive drugs such as proteins. The drug formulated as a lyophilized powder can be suspended in a polymer phase comprising a suitable encapsulating polymer dissolved in a volatile organic solvent such as methylene chloride. The suspension can be atomized by spraying into a container containing frozen ethanol overlaid with liquid nitrogen. The system can then be warmed to about -80 °C to liquefy the ethanol and extract the organic solvent from the microspheres. The hardened microspheres are collected by filtration or centrifugation and lyophilized.

### 5. The Cross-Linking Method

Another method of fabricating the nanoparticles according to an embodiment of the present invention is the cross-linking method. This procedure can be used if the selected encapsulating polymer is a thermoset polymer and therefore can be cured by cross-linking. The cross-linking method uses at least two unsaturated compounds, one of which serving as a cross-linking agent.

A solution of a water-soluble unsaturated monomer, for example, vinyl pyrrolidone (VP) in water can be prepared. The concentration of VP in the solution can be between about 5.0 and 20.0 mass %. Alternative monomers, for example, hydroxyethyl methacrylate can be used in addition to, or instead of, VP. A water-soluble cross-linking agent can then be added to the solution of VP, for example, poly(ethylene glycol diacrylate) (PEG-DA) having a weight average molecular weight of about 1,000 Daltons to form the aqueous VP/PEG-DA solution (solution IV). The concentration of PEG-DA in solution IV can be between about 5.0 and 20.0 mass %. Alternative cross-linking agents such as other diacrylates or dimethacrylates can be selected by those having ordinary skill in the art to be used in addition to, or instead of PEG-DA. A hydrophobic drug, for example, everolimus can be added to solution IV in the amount of between about 5.0 and 20.0 mass % of solution IV, forming a suspension of the drug in solution IV ("the drug suspension").

A separate solution of a photoinitiator such as 2,2-dimethoxy-2-phenyl acetophenone in VP can be made, the solution containing between about 5.0 and 20.0 mass % of the photoinitiator. Other photoinitiators, for example, dithiocarbonates or periodide can be used in the alternative. The photoinitiator solution can be added to the drug suspension to form the final blend. The ratio between the photoinitiator solution and the drug suspension can be determined by those having ordinary skill in the art.

The final blend can be added into a viscous mineral oil or silicone oil and vortexed energetically until a W-O emulsion is formed. The emulsion can then be irradiated at 360 nm wavelength using a black ray UV-lamp for about 15 to 45 seconds. As a result, VP and PEG-DA copolymerize and VP is cross-linked with PEG-DA forming VP/PEG-DA nanoparticles containing the drug. The particles can then be isolated by decanting the oil phase, washed in, e.g., acetone, and dried.

If desired, an inorganic cross-linking agent can be used. For example, an encapsulating polymer/drug suspension can be made by mixing an aqueous solution containing about 10 mass % of poly(alginate) and everolimus. The amount of the drug can be about 5 mass % of the poly(alginate) solution. The polymer/drug suspension is then combined with a solution of the cross-linking agent such as calcium chloride (CaCl₂) in de-ionized water. The amount of CaCl₂ can be about 10 mass % of the polymer/drug suspension. The polymer/drug/ CaCl₂ system can be vigorously stirred leading to cross-linking of poly(alginate) forming the cross-linked poly(alginate) nanoparticles containing the drug. The particles are then isolated by decanting, washed in de-ionized water and dried.

### Coating Construct

The nanoparticles described herein can be coated or deposited on a medical device with or without a binder polymer. The binder polymer as used herein refers to a biocompatible polymer or polymer blend which can be the same or different from the polymer that may be included in the nanoparticles.

In one embodiment, the nanoparticles can be included in a coating (i.e., a drug-delivery coating) on a medical device. Upon implantation in a subject (e.g., a patient), the nanoparticles can be released from the coating to infiltrate into the vessel or lesion so as to provide treatment to the vessel or lesion.

In some other embodiments, a coating that includes nanoparticles described herein can be formed by depositing the nanoparticles on a device followed by binding the nanoparticles to the device surface by a binder. The nanoparticles (e.g., everolimus in PEA) can be deposited on a device (e.g., stent) by a modified stereolithography technique. In this method, the device is placed in a solution comprising both the nanoparticles and a photo-reactive binder. As the solution is locally illuminated with light or invisible electromagnetic radiation with a wavelength capable of crosslinking the photoreactive binder, the binder crosslinks, trapping the nanoparticles in its matrix. The device may be rotated and translated within the bath of nanoparticles and binder such that the focus of the radiation initiates deposition of the nanoparticle-containing matrix onto the selected parts of the device. Alternatively, the nanoparticles can be deposited in channels, depots or other structural modifications capable of holding and releasing the particles. In some embodiments, the nanoparticles can be bound together by adding a blood compatible binder (e.g., a blend of D,L-PLA and high molecular weight PEG (M_{w} in the range from about 25,000 to about 40,000 Daltons) or a blend of PEA and high molecular weight PEG (M_{w} in the range from about 25,000 to about 40,000 Daltons)). A hydrophilic component such as a high molecular weight PEG can loosen up the nanoparticles in the coating once the device is deployed.

The drug can include any substance capable of exerting a therapeutic or prophylactic effect on a patient. The drug may include small molecule drugs, peptides, proteins, oligonucleotides, and combinations thereof. The drug could be designed, for example, to inhibit the activity of vascular smooth muscle cells. For example, the drug can be directed at inhibiting abnormal or inappropriate migration and/or proliferation of smooth muscle cells to inhibit restenosis. Alternatively, the drug may be designed to improve or restore functionality of endothelium, e.g. inflamed endothelium. In another application, the drug may be designed to reduce the macrophage or foam cell load of atherosclerotic disease such as vulnerable plaque.

### Biocompatible polymers

Any biocompatible polymers can be included in the nanoparticles described above and/or coatings on a device. The biocompatible polymer can be biodegradable (either bioerodable or bioabsorbable or both) or nondegradable, and can be hydrophilic or hydrophobic.

Representative biocompatible polymers include, but are not limited to, poly(ester amide), polyhydroxyalkanoates (PHA), poly(3-hydroxyalkanoates) such as poly(3-hydroxypropanoate), poly(3-hydroxybutyrate), poly(3-hydroxyvalerate), poly(3-hydroxyhexanoate), poly(3-hydroxyheptanoate) and poly(3-hydroxyoctanoate), poly(4-hydroxyalkanaote) such as poly(4-hydroxybutyrate), poly(4-hydroxyvalerate), poly(4-hydroxyhexanote), poly(4-hydroxyheptanoate), poly(4-hydroxyoctanoate) and copolymers including any of the 3-hydroxyalkanoate or 4-hydroxyalkanoate monomers described herein or blends thereof, poly(D,L-lactide), poly(L-lactide), polyglycolide, poly(D,L-lactide-co-glycolide), poly(L-lactide-co-glycolide), polycaprolactone, poly(lactide-co-caprolactone), poly(glycolide-co-caprolactone), poly(dioxanone), poly(ortho esters), poly(anhydrides), poly(tyrosine carbonates) and derivatives thereof, poly(tyrosine ester) and derivatives thereof, poly(imino carbonates), poly(glycolic acid-co-trimethylene carbonate), polyphosphoester, polyphosphoester urethane, poly(amino acids), polycyanoacrylates, poly(trimethylene carbonate), poly(iminocarbonate), polyphosphazenes, silicones, polyesters, polyolefins, polyisobutylene and ethylene-alphaolefin copolymers, acrylic polymers and copolymers, vinyl halide polymers and copolymers, such as polyvinyl chloride, polyvinyl ethers, such as polyvinyl methyl ether, polyvinylidene halides, such as polyvinylidene chloride, polyacrylonitrile, polyvinyl ketones, polyvinyl aromatics, such as polystyrene, polyvinyl esters, such as polyvinyl acetate, copolymers of vinyl monomers with each other and olefins, such as ethylene-methyl methacrylate copolymers, acrylonitrile-styrene copolymers, ABS resins, and ethylene-vinyl acetate copolymers, polyamides, such as Nylon 66 and polycaprolactam, alkyd resins, polycarbonates, polyoxymethylenes, polyimides, polyethers, poly(glyceryl sebacate), poly(propylene fumarate), poly(n-butyl methacrylate), poly(sec-butyl methacrylate), poly(isobutyl methacrylate), poly(tert-butyl methacrylate), poly(n-propyl methacrylate), poly(isopropyl methacrylate), poly(ethyl methacrylate), poly(methyl methacrylate), epoxy resins, polyurethanes, rayon, rayon-triacetate, cellulose acetate, cellulose butyrate, cellulose acetate butyrate, cellophane, cellulose nitrate, cellulose propionate, cellulose ethers, carboxymethyl cellulose, polyethers such as poly(ethylene glycol) (PEG), copoly(ether-esters) (e.g. poly(ethylene oxide-co-lactic acid) (PEO/PLA)), polyalkylene oxides such as poly(ethylene oxide), poly(propylene oxide), poly(ether ester), polyalkylene oxalates, phosphoryl choline, choline, poly(aspirin), polymers and co-polymers of hydroxyl bearing monomers such as 2-hydroxyethyl methacrylate (HEMA), hydroxypropyl methacrylate (HPMA), hydroxypropylmethacrylamide, PEG acrylate (PEGA), PEG methacrylate, 2-methacryloyloxyethylphosphorylcholine (MPC) and *n*-vinyl pyrrolidone (VP), carboxylic acid bearing monomers such as methacrylic acid (MA), acrylic acid (AA), alkoxymethacrylate, alkoxyacrylate, and 3-trimethylsilylpropyl methacrylate (TMSPMA), poly(styrene-isoprene-styrene)-PEG (SIS-PEG), polystyrene-PEG, polyisobutylene-PEG, polycaprolactone-PEG (PCL-PEG), PLA-PEG, poly(methyl methacrylate)-PEG (PMMA-PEG), polydimethylsiloxane-co-PEG (PDMS-PEG), poly(vinylidene fluoride)-PEG (PVDF-PEG), PLURONIC™ surfactants (polypropylene oxide-co-polyethylene glycol), poly(tetramethylene glycol), hydroxy functional poly(vinyl pyrrolidone), biomolecules such as collagen, chitosan, alginate, fibrin, fibrinogen, cellulose, starch, dextran, dextrin, hyaluronic acid, fragments and derivatives of hyaluronic acid, heparin, fragments and derivatives ofheparin, glycosamino glycan (GAG), GAG derivatives, polysaccharides, elastin, or combinations thereof. In some embodiments, the nanoparticles can exclude any one of the aforementioned polymers.

As used herein, the terms poly(D,L-lactide), poly(L-lactide), poly(D,L-lactide-co-glycolide), and poly(L-lactide-co-glycolide) can be used interchangeably with the terms poly(D,L-lactic acid), poly(L-lactic acid), poly(D,L-lactic acid-co-glycolic acid), or poly(L-lactic acid-co-glycolic acid), respectively.

### Biobeneficial Material

In some embodiments, the nanoparticles and/or coatings can further include a biobeneficial material. The biobeneficial material can be a polymeric material or non-polymeric material. The biobeneficial material is preferably non-toxic, non-antigenic and non-immunogenic. A biobeneficial material is one which enhances the biocompatibility of the particles or device by being non-fouling, hemocompatible, actively non-thrombogenic, or anti-inflammatory, all without depending on the release of a pharmaceutically active agent.

Representative biobeneficial materials include, but are not limited to, polyethers such as poly(ethylene glycol), copoly(ether-esters) (e.g. PEO/PLA), polyalkylene oxides such as poly(ethylene oxide), poly(propylene oxide), poly(ether ester), polyalkylene oxalates, polyphosphazenes, phosphoryl choline, choline, poly(aspirin), polymers and co-polymers of hydroxyl bearing monomers such as hydroxyethyl methacrylate (HEMA), hydroxypropyl methacrylate (HPMA), hydroxypropylmethacrylamide, poly (ethylene glycol) acrylate (PEGA), PEG methacrylate, 2-methacryloyloxyethylphosphorylcholine (MPC) and *n*-vinyl pyrrolidone (VP), carboxylic acid bearing monomers such as methacrylic acid (MA), acrylic acid (AA), alkoxymethacrylate, alkoxyacrylate, and 3-trimethylsilylpropyl methacrylate (TMSPMA), poly(styrene-isoprene-styrene)-PEG (SIS-PEG), polystyrene-PEG, polyisobutylene-PEG, polycaprolactone-PEG (PCL-PEG), PLA-PEG, poly(methyl methacrylate)-PEG (PMMA-PEG), polydimethylsiloxane-co-PEG (PDMS-PEG), poly(vinylidene fluoride)-PEG (PVDF-PEG), PLURONIC™ surfactants (polypropylene oxide-co-polyethylene glycol), poly(tetramethylene glycol), hydroxy functional poly(vinyl pyrrolidone), biomolecules such as fibrin, fibrinogen, cellulose, starch, collagen, dextran, dextrin, hyaluronic acid, fragments and derivatives of hyaluronic acid, heparin, fragments and derivatives of heparin, glycosamino glycan (GAG), GAG derivatives, polysaccharides, elastin, chitosan, alginate, silicones, PolyActive™, and combinations thereof. In some embodiments, the nanoparticles and/or coatings can exclude any one of the aforementioned polymers.

The term PolyActive™ refers to a block copolymer having flexible poly(ethylene glycol) and poly(butylene terephthalate) blocks (PEGT/PBT). PolyActive™ is intended to include AB, ABA, BAB copolymers having such segments of PEG and PBT (e.g., poly(ethylene glycol)-block-poly(butyleneterephthalate)-block poly(ethylene glycol) (PEG-PBT-PEG).

In a preferred embodiment, the biobeneficial material can be a polyether such as poly (ethylene glycol) (PEG) or polyalkylene oxide.

### Bioactive Agents

The bioactive agents forming the nanoparticles with the matrix material can be any bioactive agent, which is a therapeutic, prophylactic, or diagnostic agent. These agents can have anti-proliferative or anti-inflammmatory properties or can have other properties such as antineoplastic, antiplatelet, anti-coagulant, anti-fibrin, antithrombonic, antimitotic, antibiotic, antiallergic, and antioxidant. The agents can be cystostatic agents, agents that promote the healing of the endothelium such as NO releasing or generating agents, agents that attract endothelial progenitor cells, or agents that promote the attachment, migration and proliferation of endothelial cells (e.g., natriuretic peptide such as CNP, ANP or BNP peptide or an RGD or cRGD peptide), while quenching smooth muscle cell proliferation. Examples of suitable therapeutic and prophylactic agents include synthetic inorganic and organic compounds, proteins and peptides, polysaccharides and other sugars, lipids, and DNA and RNA nucleic acid sequences having therapeutic, prophylactic or diagnostic activities. Some other examples of the bioactive agent include antibodies, receptor ligands, enzymes, adhesion peptides, blood clotting factors, inhibitors or clot dissolving agents such as streptokinase and tissue plasminogen activator, antigens for immunization, hormones and growth factors, oligonucleotides such as antisense oligonucleotides and ribozymes and retroviral vectors for use in gene therapy. Examples of anti-proliferative agents include rapamycin and its functional or structural derivatives, 40-*O*-(2-hydroxy)ethyl-rapamycin (everolimus), and its functional or structural derivatives, and paclitaxel and its functional and structural derivatives. Examples of rapamycin derivatives include 40-epi-(N1-tetrazolyl)-rapamycin (ABT-578), 40-*O*-(3-hydroxy)propyl-rapamycin, 40-*O*-[2-(2-hydroxy)ethoxy]ethyl-rapamycin, and 40-*O*-tetrazole-rapamycin. Examples of paclitaxel derivatives include docetaxel. Examples of antineoplastics and/or antimitotics include methotrexate, azathioprine, vincristine, vinblastine, fluorouracil, doxorubicin hydrochloride (e.g. Adriamycin^{®} from Pharmacia & Upjohn, Peapack N.J.), and mitomycin (e.g. Mutamycin^{®} from Bristol-Myers Squibb Co., Stamford, Conn.). Examples of such antiplatelets, anticoagulants, antifibrin, and antithrombins include sodium heparin, low molecular weight heparins, heparinoids, hirudin, argatroban, forskolin, vapiprost, prostacyclin and prostacyclin analogues, dextran, D-phe-pro-arg-chloromethylketone (synthetic antithrombin), dipyridamole, glycoprotein IIb/IIIa platelet membrane receptor antagonist antibody, recombinant hirudin, thrombin inhibitors such as Angiomax (Biogen, Inc., Cambridge, Mass.), calcium channel blockers (such as nifedipine), colchicine, fibroblast growth factor (FGF) antagonists, fish oil (omega 3-fatty acid), histamine antagonists, lovastatin (an inhibitor of HMG-CoA reductase, a cholesterol lowering drug, brand name Mevacor^{®} from Merck & Co., Inc., Whitehouse Station, NJ), monoclonal antibodies (such as those specific for Platelet-Derived Growth Factor (PDGF) receptors), nitroprusside, phosphodiesterase inhibitors, prostaglandin inhibitors, suramin, serotonin blockers, steroids, thioprotease inhibitors, triazolopyrimidine (a PDGF antagonist), nitric oxide or nitric oxide donors, super oxide dismutases, super oxide dismutase mimetics, 4-amino-2,2,6,6-tetramethylpiperidine-1-oxyl (4-amino- TEMPO), estradiol, anticancer agents, dietary supplements such as various vitamins, and a combination thereof. Examples of anti-inflammatory agents including steroidal and non-steroidal anti-inflammatory agents include tacrolimus, dexamethasone, clobetasol, or combinations thereof. Examples of cytostatic substances include angiopeptin, angiotensin converting enzyme inhibitors such as captopril (e.g. Capoten^{®} and Capozide^{®} from Bristol-Myers Squibb Co., Stamford, Conn.), cilazapril or lisinopril (e.g. Prinivil^{®} and Prinzide^{®} from Merck & Co., Inc., Whitehouse Station, NJ). An example of an antiallergic agent is permirolast potassium. Other therapeutic substances or agents which may be appropriate include alpha-interferon, pimecrolimus, imatinib mesylate, midostaurin, bioactive RGD, and genetically engineered endothelial cells. The foregoing substances can also be used in the form af prodrugs or co-drugs thereof. The foregoing substances also include metabolites thereof and/or prodrugs of the metabolites. The foregoing substances are listed by way of example and are not meant to be limiting. Other active agents which are currently available or that may be developed in the future are equally applicable.

The dosage or concentration of the bioactive agent required to produce a favorable therapeutic effect should be less than the level at which the bioactive agent produces toxic effects and greater than the level at which non-therapeutic results are obtained. The dosage or concentration of the bioactive agent can depend upon factors such as the particular circumstances of the patient, the nature of the trauma, the nature of the therapy desired, the time over which the ingredient administered resides at the vascular site, and if other active agents are employed, the nature and type of the substance or combination of substances. Therapeutic effective dosages can be determined empirically, for example by infusing vessels from suitable animal model systems and using immunohistochemical, fluorescent or electron microscopy methods to detect the agent and its effects, or by conducting suitable in vitro studies. Standard pharmacological test procedures to determine dosages are understood by one of ordinary skill in the art.

### Examples of Implantable Device

As used herein, an implantable device may be any suitable medical substrate that can be implanted in a human or veterinary patient. Examples of such implantable devices include self-expandable stents, balloon-expandable stents, stent-grafts, grafts (e.g., aortic grafts), heart valve prostheses, cerebrospinal fluid shunts, pacemaker electrodes, catheters, and endocardial leads (e.g., FINELINE and ENDOTAK, available from Guidant Corporation, Santa Clara, CA), anastomotic devices and connectors, orthopedic implants such as screws, spinal implants, electro-stimulatory devices. The underlying structure of the device can be of virtually any design. The device can be made of a metallic material or an alloy such as, but not limited to, cobalt chromium alloy (ELGILOY), stainless steel (316L), high nitrogen stainless steel, e.g., BIODUR 108, cobalt chrome alloy L-605, "MP35N," "MP20N," ELASTINITE (Nitinol), tantalum, nickel-titanium alloy, platinum-iridium alloy, gold, magnesium, or combinations thereof. "MP35N" and "MP20N" are trade names for alloys of cobalt, nickel, chromium and molybdenum available from Standard Press Steel Co., Jenkintown, PA. "MP35N" consists of 35% cobalt, 35% nickel, 20% chromium, and 10% molybdenum. "MP20N" consists of 50% cobalt, 20% nickel, 20% chromium, and 10% molybdenum. Devices made from bioabsorbable or biostable polymers could also be used with the embodiments of the present invention.

### Method of Use

In accordance with embodiments of the invention, the nanoparticles can be released from a medical device (e.g., stent) during delivery and (in the case of a stent) expansion of the device, or thereafter, and released at a desired rate and for a predetermined duration of time at the site of implantation.

Preferably, the medical device is a stent. The stent described herein is useful for a variety of medical procedures, including, by way of example, treatment of obstructions caused by tumors in bile ducts, esophagus, trachea/bronchi and other biological passageways. A stent having the above-described coating is particularly useful for treating diseased regions of blood vessels caused by lipid deposition, monocyte or macrophage infiltration, or dysfunctional endothelium or a combination thereof, or occluded regions of blood vessels caused by abnormal or inappropriate migration and proliferation of smooth muscle cells, thrombosis, and restenosis. Stents may be placed in a wide array of blood vessels, both arteries and veins. Representative examples of sites include the iliac, renal, carotid and coronary arteries.

For implantation of a stent, an angiogram is first performed to determine the appropriate positioning for stent therapy. An angiogram is typically accomplished by injecting a radiopaque contrasting agent through a catheter inserted into an artery or vein as an x-ray is taken. A guidewire is then advanced through the lesion or proposed site of treatment. Over the guidewire is passed a delivery catheter which allows a stent in its collapsed configuration to be inserted into the passageway. The delivery catheter is inserted either percutaneously or by surgery into the femoral artery, brachial artery, femoral vein, or brachial vein, and advanced into the appropriate blood vessel by steering the catheter through the vascular system under fluoroscopic guidance. A stent having the above-described features may then be expanded at the desired area of treatment. A post-insertion angiogram may also be utilized to confirm appropriate positioning.

### Further aspects

1. A medical device comprising nanoparticles, the nanoparticles comprising a matrix, a shell, a polymer micelle, a polymerosome or combinations thereof; and a bioactive agent, wherein the matrix or shell is formed of a material selected from the group consisting of ceramic materials, bioglass, metals, and combinations thereof.
2. The medical device of aspect 1, comprising a polymeric coating that includes the nanoparticles, wherein the coating is biodegradable and the nanoparticles have a degradation time scale longer than the coating; wherein the coating is biodegradable and the nanoparticles do not begin to degrade until after released from the coating; or wherein the coating is non-biodegradable and the nanoparticles do not being to degrade until after released from the coating.
3. The medical device of aspect 1, wherein the nanoparticles comprise poly(lactic acid), poly(ester amide), or a combination thereof.
4. The medical device of aspect 1, wherein the nanoparticles comprise one or more block copolymers.
5. The medical device of aspect 1, wherein the nanoparticles comprise a polymeric shell, a polymer micelle, a polymerosome or combinations thereof.
6. The medical device of aspect 5, wherein the polymeric shell, polymer micelle, polymerosome or combinations thereof are biodegradable.
7. The medical device of aspect 1, wherein the nanoparticles comprise a matrix or shell formed of a material selected from the group consisting of ceramic materials, bioglass, metals, and combinations thereof.
8. The medical device of aspect 7, wherein the matrix or shell is biodegradable.
9. The medical device of aspect 1, wherein the matrix or shell comprises manganese (Mn), gold (Au), iron, or combinations thereof.
10. The medical device of aspect 1, wherein the nanoparticles comprise a biopolymer.
11. The medical device of aspect 10, wherein the biopolymer comprises a material selected from the group consisting of chitosan, silk elastin, poly(acrylic acid) (PAA), lectin-conjugated polymers, lipid- or cholesterol-conjugated polymers or copolymers, and combinations thereof.
12. The medical device of aspect 1, wherein the nanoparticles comprise a surface modified by grafting of polymers, peptides, proteins, or combinations thereof.
13. The medical device of aspect 1, wherein the nanoparticles comprise a surface modified by a non-immunogenic polymer, an adhesion molecule, or a combination thereof.
14. The medical device of aspect 13, wherein the non-immunogenic polymer is polyethylene glycol (PEG).
15. The medical device of aspect 13, wherein the adhesion molecule is an RGD peptide, cyclic RGD peptide, RGD mimetics, or combinations thereof.
16. The medical device of aspect 13, wherein the adhesion molecule is a peptide with affinity to a vasculature surface molecule.
17. The medical device of aspect 1, comprising a drug-delivery coating that comprises the nanoparticles, wherein the nanoparticles are capable of being released from the coating and infltrating into a vessel or lesion so as to provide treatment to the vessel or lesion.
18. The medical device of aspect 1, wherein the nanoparticles are deposited on the device by a stereolithography technique.
19. The medical device of aspect 1, wherein the nanoparticles are bound together by a blood compatible binder, and wherein the binder comprises at least one of a biocompatible polymer and a high molecular weight PEG.
20. The medical device of aspect 12, wherein the polymers comprise poly(D,L-lactic acid), poly(ester amide), or a combination thereof.
21. The medical device of aspect 1, wherein the nanoparticles are deposited in channels or depots of the device.
22. The medical device of aspect 21, wherein the nanoparticles are bound together by a blood compatible binder, and wherein the binder comprises at least one of a biocompatible polymer and a high molecular weight PEG.
23. The medical device of aspect 22, wherein the biocompatible polymer is poly(D,L-lactic acid), poly(ester amide), or a combination thereof.
24. The medical device of aspect 1, comprising a polymeric coating that includes the nanoparticles, wherein the nanoparticles comprise a polymeric shell, a polymer micelle, a polymerosome or combinations thereof, and wherein the polymeric coating degrades faster than the polymeric shell, the polymer micelle, the polymerosome or combinations thereof.
25. The medical device of aspect 1, wherein the medical device comprises a biodegradable body portion, wherein the nanoparticles are embedded within the biodegradable body portion, and wherein the nanoparticles either have degradation time scales longer than the biodegradable body portion or do not begin to degrade until released from the biodegradable body portion.
26. The medical device of aspect 25, wherein the medical device is a stent.
27. The medical device of aspect 25, wherein the biodegradable body portion shields the nanoparticles from degradation.
28. The medical device of aspect 25, wherein at least one of the nanoparticles or the biodegradable body portion is capable of enzymatic degradation.
29. The medical device of aspect 1, having a porous structure or micro depots on the surface, wherein the structure or surface is metal, ceramic, carbon, plastic, polymeric or combinations thereof, and wherein the nanoparticles are loaded pre-deployment into and released from the porous structure or the micro depots on the surface.
30. The medical device of aspect 29, wherein the porous structure is a nanoporous structure.
31. The medical device of aspect 1, wherein the device is a stent.
32. The medical device of aspect 31, wherein the bioactive agent is selected from the group consisting of paclitaxel, docetaxel, estradiol, nitric oxide donors, super oxide dismutases, super oxide dismutases mimics, 4-amino-2,2,6,6-tetramethylpiperidine-1-oxyl (4-amino-TEMPO), tacrolimus, dexamethasone, rapamycin, rapamycin derivatives, 40-*O-*(2-hydroxy)ethyl-rapamycin (everolimus), 40-*O*-(3-hydroxy)propyl-rapamycin, 40-*O*-[2-(2-hydroxy)ethoxy]ethyl-rapamycin, and 40-*O*-tetrazole-rapamycin, 40-epi-(N1-tetrazolyl)-rapamycin (ABT-578), clobetasol, pimecrolimus, imatinib mesylate, midostaurin, prodrugs thereof, co-drugs thereof, and a combination thereof.
33. A method of treating a disorder in a patient comprising implanting in the patient the medical device of aspect 32, wherein the disorder is selected from the group consisting of atherosclerosis, thrombosis, restenosis, hemorrhage, vascular dissection or perforation, vascular aneurysm, vulnerable plaque, chronic total occlusion, claudication, anastomotic proliferation for vein and artificial grafts, bile duct obstruction, ureter obstruction, tumor obstruction, and combinations thereof.

## Claims

1. A medical device, comprising:
(a) nanoparticles, which nanoparticles include a bioactive agent and comprise a matrix formed of a polymer and a shell formed of a biodegradable metal, wherein the bioactive agent is a drug which is loaded in said matrix; and
(b) a channelled surface or a biodegradable body portion;
wherein
when the medical device comprises said channelled surface, the nanoparticles are deposited within channels capable of releasing said nanoparticles,
and
when the medical device comprises said biodegradable body portion, the nanoparticles are embedded within the biodegradable body portion.

2. A medical device according to claim 1 wherein the shell comprises iron.

3. A medical device according to claim 1 or claim 2 which comprises said biodegradable body portion, wherein the nanoparticles have degradation time scales longer than the biodegradable body portion or do not begin to degrade until released from the biodegradable body portion.

4. A medical device according to claim 3, wherein the biodegradable body portion shields the nanoparticles from degradation.

5. A medical device according to claim 1 or claim 2, wherein the biodegradable body portion comprises a polymeric matrix and the nanoparticles are embedded within said matrix and the nanoparticles have degradation time scales shorter than said matrix.

6. A medical device according to claim 3 wherein the biodegradable body portion is capable of enzymatic degradation.

7. A medical device according to claim 1 or claim 2 having a channelled surface, wherein the surface is carbon, metallic, ceramic, plastic or polymeric.

8. A medical device according to claim 1 or claim 2, wherein the nanoparticles comprise poly(lactic acid), poly(ester amide), or a combination thereof

9. A medical device according to claim 1 or claim 2, wherein the nanoparticles comprise one or more block co-polymers.

10. A medical device according to claim 1 or claim 2, wherein the nanoparticles comprise a surface modified by grafting of polymers, peptides, proteins, or combinations thereof, or wherein the nanoparticles comprise a surface modified by a non-immunogenic polymer, an adhesion molecule, or a combination thereof.

11. A medical device according to claim 1 or claim 2, wherein the nanoparticles are bound together by a blood compatible binder, which is a blend of poly(D,L-lactic acid) and high molecular weight poly(ethylene glycol) or a blend of poly(ester amide) and high molecular weight poly(ethylene glycol), wherein said high molecular weight poly(ethylene glycol) has a molecular weight of from 25,000 to 40,000 Daltons.

12. A medical device according to claim 1 or claim 2, wherein the nanoparticles comprise a biopolymer, wherein the biopolymer comprises a material selected from the group consisting of chitosan, silk elastin, poly(acrylic acid) (PAA), lectin-conjugated polymers, lipid- or cholesterol-conjugated polymers, and combinations thereof.

13. A medical device according to claim 10 wherein the non-immunogenic polymer is polyethylene glycol (PEG), wherein the adhesion molecule is an RGD peptide, cyclic RGD peptide, RGD mimetics, or combinations thereof, or wherein the adhesion molecule is a peptide with affinity to a vasculature surface molecule.

14. A medical device according to claim 11 which comprises said channelled surface.

15. A medical device according to any one of the preceding claims, wherein the bioactive agent is selected from the group consisting of paclitaxel, docetaxel, estradiol, nitric oxide donors, super oxide dismutases, super oxide dismutases mimics, 4-amino-2,2,6,6-tetramethylpiperidine-1-oxyl (4-amino-TEMPO), tacrolimus, dexamethasone, rapamycin, 40-*O*-(2-hydroxy)ethyl-rapamycin (everolimus), 40-O-(3-hydroxy)propyl-rapamycin, 40-O-[2-(2-hydroxy)ethoxy]ethyl-rapamycin, 40-O-tetrazole-rapamycin, 40-epi-(N1-tetrazolyl)-rapamycin (ABT-578), clobetasol, pimecrolimus, imatinib mesylate, midostaurin, and combinations thereof.

## Patentansprüche

1. Medizinische Vorrichtung, die Folgendes umfasst:
(a) Nanopartikel, wobei die Nanopartikel ein bioaktives Mittel beinhalten sowie eine aus einem Polymer gebildete Matrix und eine aus einem biologisch abbaubaren Metall gebildete Schale umfassen, wobei das bioaktive Mittel ein Medikament ist, das in die Matrix geladen ist, und
(b) eine kanalisierte Oberfläche oder einen biologisch abbaubaren Körperabschnitt,
wobei
wenn die medizinische Vorrichtung die kanalisierte Oberfläche umfasst, die Nanopartikel in Kanälen, die die Nanopartikel freisetzen können, abgesetzt sind,
und
wenn die medizinische Vorrichtung den biologisch abbaubaren Körperabschnitt umfasst, die Nanopartikel im biologisch abbaubaren Körperabschnitt eingebettet sind.

2. Medizinische Vorrichtung nach Anspruch 1, wobei die Schale Eisen umfasst.

3. Medizinische Vorrichtung nach Anspruch 1 oder Anspruch 2, die den biologisch abbaubaren Körperabschnitt umfasst, wobei die Nanopartikel längere Abbauzeitskalen haben als der biologisch abbaubare Körperabschnitt oder erst beginnen abzubauen, wenn sie vom biologisch abbaubaren Körperabschnitt freigesetzt werden.

4. Medizinische Vorrichtung nach Anspruch 3, wobei der biologisch abbaubare Körperabschnitt die Nanopartikel gegenüber dem Abbau abschirmt.

5. Medizinische Vorrichtung nach Anspruch 1 oder Anspruch 2, wobei der biologisch abbaubare Körperabschnitt eine polymerische Matrix umfasst und die Nanopartikel in die Matrix eingebettet sind und die Nanopartikel kürzere Abbauzeitskalen haben als die Matrix.

6. Medizinische Vorrichtung nach Anspruch 3, wobei der biologisch abbaubare Körperabschnitt zum enzymatischen Abbau in der Lage ist.

7. Medizinische Vorrichtung nach Anspruch 1 oder Anspruch 2 mit einer kanalisierten Oberfläche, wobei die Oberfläche Kohlenstoff, metallisch, keramisch, Kunststoff oder polymerisch ist.

8. Medizinische Vorrichtung nach Anspruch 1 oder Anspruch 2, wobei die Nanopartikel Poly(milchsäure), Poly(esteramid) oder eine Kombination davon umfassen.

9. Medizinische Vorrichtung nach Anspruch 1 oder Anspruch 2, wobei die Nanopartikel ein oder mehrere Blockcopolymere umfassen.

10. Medizinische Vorrichtung nach Anspruch 1 oder Anspruch 2, wobei die Nanopartikel eine durch Pfropfen von Polymeren, Peptiden, Proteinen oder Kombinationen davon modifizierte Oberfläche umfassen oder wobei die Nanopartikel eine durch ein nicht-immunogenes Polymer, ein Adhäsionsmolekül oder eine Kombination davon modifizierte Oberfläche umfasst.

11. Medizinische Vorrichtung nach Anspruch 1 oder Anspruch 2, wobei die Nanopartikel durch ein blutkompatibles Bindemittel, bei dem es sich um ein Gemisch aus Poly(D,L-Milchsäure) sowie Poly(ethylenglycol) von hohem Molekulargewicht oder ein Gemisch aus Poly(esteramid) sowie Poly(ethylenglycol) von hohem Molekulargewicht handelt, miteinander verbunden sind, wobei das Poly(ethylenglycol) von hohem Molekulargewicht ein Molekulargewicht von 25.000 bis 40.000 Dalton hat.

12. Medizinische Vorrichtung nach Anspruch 1 oder Anspruch 2, wobei die Nanopartikel ein Biopolymer umfassen, wobei das Biopolymer ein Material umfasst, das aus der aus Chitosan, Seidenelastin, Poly(acrylsäure) (PAA), lektin-konjugierten Polymeren, lipid- oder cholesterinkonjugierten Polymeren bestehenden Gruppe und Kombinationen davon ausgewählt ist.

13. Medizinische Vorrichtung nach Anspruch 10, wobei das nicht-immunogene Polymer Polyethylenglycol (PEG) ist, wobei das Adhäsionsmolekül ein RGD-Peptid, zyklisches RGD-Peptid, RGD-Mimetika oder Kombinationen davon ist oder wobei das Adhäsionsmolekül ein Peptid mit Affinität zu einem Gefäßsystem-Oberflächenmolekül ist.

14. Medizinische Vorrichtung nach Anspruch 11, die die kanalisierte Oberfläche umfasst.

15. Medizinische Vorrichtung nach einem der vorstehenden Ansprüche, wobei das bioaktive Mittel aus der aus Paclitaxel, Docetaxel, Estradiol, Stickstoffoxid-Donoren, Superoxiddismutasen, Superoxiddismutase-Imitatoren, 4-Amino-2,2,6,6-Tetramethylpiperidine-1-Oxyl (4-Amino-TEMPO), Tacrolimus, Dexamethason, Rapamycin, 40-O-(2-Hydroxy)ethyl-Rapamycin (Everolimus), 40-O-(3-Hydroxy)propyl-Rapamycin, 40-O-[2-(2-Hydroxy)ethoxy]ethyl-Rapamycin, 40-O-Tetrazole-Rapamycin, 40-Epi-(N1-Tetrazolyl)-Rapamycin (ABT-578), Clobetasol, Pimecrolimus, Imatinibmesylat, Midostaurin und Kombinationen davon ausgewählt ist.

## Revendications

1. Dispositif médical, comprenant :
(a) des nanoparticules, lesquelles nanoparticules incluent un agent bioactif et comprennent une matrice formée d'un polymère et une enveloppe formée d'un métal biodégradable, dans lequel l'agent bioactif est un médicament qui est chargé dans ladite matrice ; et
(b) une surface canalisée ou une portion de corps biodégradable ;
dans lequel
lorsque le dispositif médical comprend ladite surface canalisée, les nanoparticules sont déposées à l'intérieur de canaux capables de libérer lesdites nanoparticules, et
lorsque le dispositif médical comprend ladite portion de corps biodégradable, les nanoparticules sont incorporées à l'intérieur de la portion de corps biodégradable.

2. Dispositif médical selon la revendication 1, dans lequel l'enveloppe comprend du fer.

3. Dispositif médical selon la revendication 1 ou la revendication 2, qui comprend ladite portion de corps biodégradable, dans lequel les nanoparticules présentent des échelles de temps de dégradation plus longues que celle de la portion de corps biodégradable ou ne commencent pas à se dégrader avant d'être libérées de la portion de corps biodégradable.

4. Dispositif médical selon la revendication 3, dans lequel la portion de corps biodégradable protège les nanoparticules de la dégradation.

5. Dispositif médical selon la revendication 1 ou la revendication 2, dans lequel la portion de corps biodégradable comprend une matrice polymérique et les nanoparticules sont incorporées à l'intérieur de ladite matrice et les nanoparticules présentent des échelles de temps de dégradation plus courtes que celle de ladite matrice.

6. Dispositif médical selon la revendication 3, dans lequel la portion de corps biodégradable est capable de dégradation enzymatique.

7. Dispositif médical selon la revendication 1 ou la revendication 2, possédant une surface canalisée, dans lequel la surface est en carbone, est métallique, en céramique, plastique ou est polymérique.

8. Dispositif médical selon la revendication 1 ou la revendication 2, dans lequel les nanoparticules comprennent : poly(acide lactique), poly(ester-amide), ou une association de ceux-ci.

9. Dispositif médical selon la revendication 1 ou la revendication 2, dans lequel les nanoparticules comprennent un ou plusieurs copolymères à blocs.

10. Dispositif médical selon la revendication 1 ou la revendication 2, dans lequel les nanoparticules comprennent une surface modifiée par greffage de : polymères, peptides, protéines, ou associations de ceux-ci, ou dans lequel les nanoparticules comprennent une surface modifiée par un polymère non immunogène, une molécule d'adhésion, ou une association de ceux-ci.

11. Dispositif médical selon la revendication 1 ou la revendication 2, dans lequel les nanoparticules sont liées ensemble par un liant à compatibilité sanguine, qui est un mélange de poly(D,L-acide lactique) et de poly(éthylène glycol) de haut poids moléculaire ou un mélange de poly(ester-amide) et de poly(éthylène glycol) de haut poids moléculaire, dans lequel ledit poly(éthylène glycol) de haut poids moléculaire possède un poids moléculaire de 25000 à 40000 Daltons.

12. Dispositif médical selon la revendication 1 ou la revendication 2, dans lequel les nanoparticules comprennent un biopolymère, dans lequel le biopolymère comprend un matériau sélectionné parmi le groupe constitué de : chitosane, élastine de soie, poly(acide acrylique) (PAA), polymères conjugués par lectine, polymères conjugués par lipide ou cholestérol, et associations de ceux-ci.

13. Dispositif médical selon la revendication 10 dans lequel le polymère non immunogène est du polyéthylène glycol (PEG), dans lequel la molécule d'adhésion est un peptide RGD, un peptide RGD cyclique, un mimétique RGD, ou des associations de ceux-ci, ou dans lequel la molécule d'adhésion est un peptide avec affinité à une molécule de surface de vasculature.

14. Dispositif médical selon la revendication 11, qui comprend ladite surface canalisée.

15. Dispositif médical selon l'une quelconque des revendications précédentes, dans lequel l'agent bioactif est sélectionné parmi le groupe constitué de : paclitaxel, docétaxel, estradiol, donneurs d'oxyde nitrique, superoxydes dismutases, imitations de superoxydes dismutases, 4-amino-2,2,6,6-tétraméthylpipéridine-1-oxyl (4-amino-TEMPO), tacrolimus, dexaméthasone, rapamycine, 40-O-(2-hydroxy)éthyl-rapamycine (évérolimus), 40-O-(3-hydroxy)propyl-rapamycine, 40-O-[2-(2-hydroxy)éthoxy]éthyl-rapamycine, 40-O-tétrazole-rapamycine, 40-épi-(N1-tétrazolyl)-rapamycine (ABT-578), clobétasol, pimecrolimus, mésylate d'imatinib, midostaurine, et associations de ceux-ci.
